Europäisches Patentamt

European Patent Office

Office européen des brevets

(11) Publication number: **0 118 180**
**B1**

# EUROPEAN PATENT SPECIFICATION

(45) Date of publication of patent specification: **25.07.90**

(51) Int. Cl.⁵: **A 61 K 7/06**

(21) Application number: **84300422.7**

(22) Date of filing: **25.01.84**

(54) Cosmetic.

(30) Priority: **02.02.83 GB 8302838**

(43) Date of publication of application:
**12.09.84 Bulletin 84/37**

(45) Publication of the grant of the patent:
**25.07.90 Bulletin 90/30**

(84) Designated Contracting States:
**AT BE CH DE FR GB IT LI LU NL SE**

(56) References cited:
**DE-A-2 532 780**
**GB-A-2 103 196**
**GB-A-2 113 116**
**US-A-4 000 317**

**PATENTS ABSTRACTS OF JAPAN, vo.7, nr.118,**
**May 21, 1983, page 1263 C-167**

**The file contains technical information**
**submitted after the application was filed and**
**not included in this specification**

(73) Proprietor: **BEECHAM GROUP PLC**
**Beecham House Great West Road**
**Brentford Middlesex TW8 9BD (GB)**

(72) Inventor: **Fairhurst, Edgar**
**48 Warenne Road Fetcham**
**Leatherhead Surrey (GB)**
Inventor: **Keyser, Anne Marion**
**West Riddens Anstye**
**Haywards Heath Sussex (GB)**

(74) Representative: **Russell, Brian John et al**
**Beecham Pharmaceuticals Great Burgh Yew**
**Tree Bottom Road**
**Epsom Surrey KT18 5XQ (GB)**

**Description**

The present invention relates to a new use of salanised adsorbents and in particular to antiseborrheic compositions containing adsorbents such as silanised silica gel.

US—A—4 000 317 discloses a skin care composition comprising pyrogenic (fumed) colloidal silica in an aqueous alcoholic medium.

DE—A—2 532 780 discloses a pulverous dry shampoo composition containing a high percentage of silica gel (which may optionally be silanized) for cleaning greasy hair. After application to the hair, the composition is brushed out.

Silanised silica gel is a known adsorbent and may be produced either by

a) treating fine particles of silica gel with a reactive silane thereby replacing many of the hydroxy groups with substituted siloxy groups, or

b) polymerising a suitable siloxane monomer in the presence of fine particles of silica gel thereby coating the particles with a polysiloxane.

Reactive silanes useful in methods (a) above include alkyl, aryl especially phenyl and aralkyl especially benzyl halosilanes and -silazanes and alkyl, aryl especially phenyl and aralkyl especially benzyl alkoxysilanes and -silazanes.

Particular examples of such silanes include

dimethyl monochlorosilane
dimethyl dichlorosilane
trimethylchlorosilane
hexamethyl disilazane
trichloro-octadecylsilane

Suitable siloxane monomers for use in method (b) above include hydrogen polysiloxanes such as methyl hydrogen polysiloxane and alkyl, aryl and aralkyl cyclotetrasiloxanes, especially octamethylcyclotetrasiloxane. Polymerisation may be effected by heating or by chemical catalysis.

It has now been found that silanised adsorbents, in particular silanised silica gel, have advantageous properties as adsorbents of sebum enabling treatment of seborrhea by removal of sebum from the skin surface and hair.

As used herein the terms 'seborrhea' and 'seborrheic conditions' refer to a range of conditions from simple cosmetic 'greasy' skin and hair to clinical seborrhea which is often associated with pimpling or acne including dry acne. In all cases of seborrhea a major contributory factor is excessive secretion of sebum which accumulates in and on the stratum corneum and hair and/or in sebaceous follicles. Some contribution to the abnormally high lipid accumulation comes from the lipids exuded from differenciating skin cells. The use of silanised adsorbents is effective in all seborrheic conditions.

It is believed that silanised adsorbents not only remove the surface lipids but also drain the sebum from the lipid reservoirs in the stratum corneum and sebaceous follicles. The latter effect is important since by draining the reservoirs of sebum the rate of regreasing of the skin surface may be reduced with commensurate improvement in the appearance of the skin.

Silanised adsorbents also have an instant cosmetic matting effect thereby immediately improving the appearance of greasy skin. As they do not adsorb water, they do not have a tendency to cake and they remain effective despite perspiration and high ambient humidity.

Accordingly the present invention provides a skin- or hair-care composition for alleviating seborrheic conditions, in the form of a roll-on formulation, gel, cream, lotion, or stick, comprising 0.1 to 5% w/w of silanised silica gel having a mean aggregate particle sie of 0.1 to $20 \times 10^{-6}$m, and an inert diluent or carrier therefor.

Suitably the 'particles' consist of aggregates of very fine 'primary particles' of much smaller size.

Preferably the silanised adsorbent is applied to the skin at regular intervals, such as twice a day.

Whilst the salanised adsorbent may be applied directly to the skin or hair, preferably it is applied in the form of a skin or hair-care composition.

Suitably the composition is presented in a conventional skin or hair-care form, for instance as a roll-on formulation, gel, cream, lotion or stick, and will contain conventional carriers and diluents known to be suitable for each particular presentation.

Preferably the composition comprises 0.1 to 5% w/w of the silanised adsorbent, most preferably 0.1 to 2%.

The composition may also comprise optional accessory ingredients such as perfume, colouring and preserving agents, keratolytics, antibacterials and antimicrobial agents such as Irgasan or chlorhexidine.

When silanised silica gel is used as the silanised adsorbent in a skin- or hair-care composition as described above the adsorbent may be incorporated without difficulty in non-aqueous media. However, to achieve homogeneity in products having an aqueous base it is advantageous, though not always essential, to include at least 2% w/w of a lower alkanol such as isopropanol or, preferably, ethanol. More preferably the product contains 10 to 50% w/w of an alkanol.

Preferably ethanol is included in any of the aforementioned skin- and hair-care compositions at from 10 to 50% w/w, more preferably at about 30% w/w to impart astringency and a cooling effect.

The following Examples illustrate the invention but are not intended to limit the scope of the invention in any way.

2

Example 1
Oil free lotion

This lotion would be applied to the skin, usually the face, on a regular (1—3 times per day) basis. It would be left on the skin between washing or cleansing.

| Ingredient | % w/w |
|---|---|
| Carbopol 941 (cosmetic thickener) | 0.20 |
| Ethanol | 30 |
| Polymist B6 (cosmetic matting agent) | 2 |
| Silanised silica gel | 2 |
| Polyethylene glycol 7 glyceryl cocoate | 2 |
| Di-isopropanolamine | 0.12 |
| Deionised water to | 100 |

Example 2
Ethanol free lotion

This lotion may be used where maximum binding of lipid to the active ingredient is required and some emolliency is not objectionable.

| Ingredient | % w/w |
|---|---|
| Sorbitan stearate | 2 |
| Polysorbate | 3.5 |
| Mineral oil | 15 |
| Cetostearylalcohol | 2.5 |
| Silanised silica gel | 2 |
| Preservative | 0.2 |
| Deionised water to | 100 |

Example 3
Wip-on, wipe-off cleansing lotion

| Ingredient | % w/w |
|---|---|
| Ethanolamine lauryl ether sulphate | 1.0 |
| Ethanol | 10 |
| Carbopol 941 | 0.2 |
| Silanised silica gel | 2.0 |
| Water to | 100 |

Example 4
Cleansing lotion

| Ingredient | % w/w |
|---|---|
| Primal ICS-1 (suspending agent) | 1.67 |
| Triethanolamine | 1 |
| Ethanol | 30 |
| Silanised silica gel | 0.5 |
| Water (deionised) to | 100 |

The lotion may be used as a liquid or impregnated into cotton fabric or similar material for use as swabs.

**Claims**

1. A skin- or hair-care composition for alleviating seborrheic conditions, in the form of a roll-on formulation, gel, cream, lotion, or stick comprising 0.1 to 5% w/w of silanised silica gel having a mean aggregate particle size of 0.1 to $20 \times 10^{-6}$ m, and an inert diluent or carrier therefor.

2. A composition as claimed in Claim 1, in the form of a skin-care composition.

3. A composition as claimed in Claim 2, in the form of a leave-on lotion.

4. A composition as claimed in Claim 2, in the form of a lotion impregnated into swabs.

3

5. A composition as claimed in any preceding claim, comprising at least 2% w/w of the total composition of ethanol.

6. A method for alleviating greasy skin or hair, which method comprises applying a composition as claimed in any preceding claim, to the skin or hair.

7. Silanised silica gel as defined in Claim 1, or a composition as claimed in any preceding claim, for use in alleviating clinical seborrhoea, including acne.

8. Use of silanised silica gel as defined in Claim 1, or a composition as claimed in any one of Claims 1 to 5, for the manufacturing of a medicament for alleviating clinical seborrhea, including acne.

**Patentansprüche**

1. Eine Haut- oder Haarpflegezusammensetzung zur Linderung von Seborrhöe-Zuständen, in Form einermittels eines Rollstiftes auftragbaren Formulierung(roll-on formulation), eines Gels, einer Creme, einer Lotion oder eines Stiftes, enthaltend 0,1 bis 5 Gewichtsprozent silanisiertes Siliciumdioxidgel mit einer mittleren Aggregatteilchengröße von 0,1 bis $20 \times 10^{-6}$ m und ein inertes Verdünngsmittel oder einen Träger hierfür.

2. Eine Zusammensetzung wie in Anspruch 1 beansprucht, in Form einer Hauptflegezusammensetzung.

3. Eine Zusammensetzung wie in Anspruch 2, beansprucht, in Form einer Lotion, welche auf der Haut helassen wird.

4. Eine Zusammensetzung wie un Anspruch 2, beansprucht, in Form einer Lotion, mit welcher Tupfer getränkt sind.

5. Eine Zusammensetzung wie in einem der vorstehenden Ansprüche beansprucht, enthaltend mindestens 2 Gewichtsprozent Äthanol, bezogen auf die Gesamtzusammensetzung.

6. Ein Verfahren zur Linderung des Erscheinungsbildes fettiger Haut oder fettigen Haares, welches das Auftragen einer Zusammensetzung, wie in einem der vorstehenden Ansprüche beansprucht, auf die Haut oder das Haar umfaßt.

7. Silanisiertes Siliciumdioxidgel, wie in Anspruch 1 definiert, oder eine Zusammensetzung, wie in einem der vorstehenden Ansprüche beansprucht, zur Vurwendung bei der Linderung klinischer Seborrhöe einschließlich Akne.

8. Verwendung von silanisiertem Siliciumdioxidgel, wie in Anspruch 1 definiert, oder einer Zusammensetzung wie in einem der Ansprüche 1 bis 5 beansprucht, zur Herstellung eines Medikaments für die Linderung klinischer Seborrhöe einschließlich Akne.

**Revendications**

1. Composition de soin pour la peau ou les cheveux pour améliorer des états séborrhéiques, sous la forme de formulation à appliquer avec une bille, gel, crème, lotion ou bâton, caractérisée en ce qu'elle comprend de 0,1 à 5% en p/p de gel de silice silanisé ayant une dimension particulaire d'agrégat moyenne de 0,1 à $20.10^{-6}$ m, et un diluant ou un support inerte pour celui-ci.

2. Composition suivant la revendication 1, caractérisée en ce qu'elle est sous la forme d'une composition de soin pour la peau.

3. Composition suivant la revendication 2, caractérisée en ce qu'elle est sous la forme d'une lotion à laisser sur la peau.

4. Composition suivant la revendication 2, caractérisé en ce qu'elle est sous la forme de lotion imprégnée dans des tempons.

5. Composition suivant l'une quelconque des revendications 1 à 4, caractérisée en ce qu'elle comprend au moins 2% en p/p d'éthanol par rapport à la composition totale.

6. Méthode pour améliorer une peau ou des cheveux graisseux, caractérisée en ce qu'elle comprend l'application sur la peau ou les cheveux, d'une composition suivant l'une quelconque des revendications 1 à 5.

7. Gel de silice silanisé défini dans la revendication 1, ou composition suivant l'une quelconque des revendications 1 à 5, utile pour améliorer une séborrhée clinique, y comprise l'acné.

8. Utilisation d'un gel de silice silanisé suivant la revendication 1 ou d'une composition suivant l'une quelconque des revendications 1 à 5, pour la fabrication d'un médicament pour améliorer une séborrhée clinique, y compris l'acné.